Europäisches Patentamt

European Patent Office

Office européen des brevets

(11) Publication number: **0 001 888**
A1

## EUROPEAN PATENT APPLICATION

(21) Application number: **78300478.1**

(22) Date of filing: **10.10.78**

(51) Int. Cl.²: **A 61 L 13/00**
**A 61 L 1/00**

(30) Priority: **31.10.77 US 847089**

(43) Date of publication of application:
**16.05.79 Bulletin 79/10**

(84) Designated contracting states:
**BE CH DE FR GB LU NL SE**

(71) Applicant: **FISONS CORPORATION**
**2 Preston Court**
**Bedford Massachusetts(US)**

(72) Inventor: **McPherson, Thomas C**
**25 Norwich Road**
**Nashua New Hampshire 83060(US)**

(74) Representative: **Craig, Christopher Bradberry et al,**
**Fisons Limited Fison House Princes Street**
**Ipswich IP1 1QH(GB)**

(54) Sterilising composition and methods for its manufacture and use.

(57) A sterilising solution for sterilizing contact lenses, particularly soft hydrogel-type contact lenses, which sterilizing solution contains, as the active bacteriostatic ingredients thereof, a solution of sodium chloride in combination with alcohol, particularly ethyl alcohol, and, optionally, a protein-denaturing amount of urea. Soft contact lenses immersed in and treated with such sterilizing solutions are sterilized rapidly and effectively in a short period of time against a variety of bacteria.

BA 2/77

Sterilising composition and methods for its manufacture and use

Contact lenses composed of plastics material have been adopted increasingly for general use. Such adoption and use have required the use of cleaning, sterilizing and/or storing solutions. After removal from the eye, the lens should be cleaned physically to remove foreign matter, such as tear-stain deposits and other accumulated foreign matter. In addition, such lenses should be sterilized against micro-organisms prior to use. Typically, the user periodically removes the contact lens from the eye and effects cleaning, sterilizing and storing in a solution for a period of time, e.g. overnight.

Contact lenses have been composed of hard and soft plastics material. Hard plastic-type contact lenses, composed, for example, of conventional hard polymethacrylate polymers, have long been in use. Soft contact lenses, which may be of the hydrophilic or hydrophobic type, have been developed and adopted more recently. Such soft lenses may be composed either of hydrophobic-type polymers or hydrophilic-type polymers. The hydrophobic contact lenses often are based upon a polysiloxane polymer, such as cross-linked dimethyl polysiloxane. The soft hydrophilic-type contact lenses generally are composed of a polyhydroxylated polymer like, alkyl methacrylate, which has been cross-linked sparingly to form a polymeric hydrogel-type structure. Such lenses are elastic, soft, transparent hydrogels containing, and permitting the lenses to absorb, a substantial amount of water.

While the soft, hydrophilic, hydrogel-type contact lenses are particularly useful, they have created difficult cleaning, sterilizing and storing problems, due in part to the hydrogel-type structure of such soft contact lenses, and the ability of such structures to absorb and even to concentrate ingredients from the cleaning, sterilizing and storing solutions. Such soft contact lenses typically are composed of a three-dimensional hydrophylic polymer, for example polyhydroxyethyl-methacrylate.

A wide variety of cleaning, sterilizing and/or storing solutions have been suggested for use with contact lenses, and particularly with the hydrogel-type soft contact lenses. Some solutions have contained quaternary-ammonium compounds or other antimicrobial agents, such as chlorhexidene (see US Patent No 3,689,673). Some sterilizing solutions for contact lenses have included specific alcohols, such as phenylethyl alcohol (see US Patent No 3,954,965) and polyvinyl alcohol (see US Patent Nos 3,183,152; 3,549,747; and 3,639,576).

Other contact-lens-treating solutions have employed various active ingredients, such as oxygen-releasing salts (see US Patent 3,873,696), phosphates (see US Patents 3,240,709 and 2,547,653), cellulosic components, such as methyl cellulose (see US Patent 3,171,752), and various block copolymers of polyethylene or polypropylene glycols (see US Patents 3,954,644 and 3,882,036). Such compositions typically contain small amounts of a saline solution, together with the active ingredients, in order to provide an isotonic aqueous cleaning and sterilizing solution.

Such contact-lens-treating solutions have not proven to be wholly satisfactory in use, and typically vary in effectiveness against a wide range of micro-organisms often found particularly with the soft contact lenses, or contain rather exotic, potentially toxic or irritating ingredients which may limit their use. It is, therefore, desirable to provide a simple, safe and effective aqueous sterilizing solution containing non-irritating and non-toxic substances which would be effective against the variety of mixed micro-organisms typically found on contact lenses, with a rapid sterilizing effectiveness such that the sterilization could be accomplished easily overnight; for example, 8 hours and preferably even in shorter periods of time.

According to the invention there is provided an aqueous mixture of a sterilising proportion of two or more of a physiologically acceptable salt, a physiologically acceptable alcohol and a physiologically acceptable protein-solubilizing agent.

According to the invention there is also provided a method for the disinfection and storage of an object, for example medical apparatus, notably contact lenses, and in particular soft contact lenses, which method comprises contacting the object with an aqueous composition containing a sterilizing proportion of at least two of a physiologically acceptable salt, a physiologically acceptable alcohol and a physiologically acceptable protein-solubilizing agent for a time sufficient to sterilize the object.

The composition may consist essentially of water, sodium chloride and ethanol.

In particular the invention concerns an aqueous sterilizing solution which comprises in combination a saturated or substantially saturated saline solution with ethyl alcohol, and preferably and optionally a protein-denaturing agent, such as urea, and to a method of employing such a solution in the sterilizing of soft, hydrophilic contact lenses.

If desired the sterilizing solution may contain more than one compound selected from each group of compounds which go to make up the composition. Thus the composition may contain two or more physiologically acceptable salts, two or more physiologically acceptable alcohols, and/or two or more physiologically acceptable protein-solubilizing agents.

It has now surprisingly been found that, while a saturated or substantially saturated aqueous sodium chloride solution at about 33% weight/volume sodium chloride is an effective antimicrobial solution, and that, while both urea and ethyl alcohol at 8 molar and 20% by volume, respectively, are effective bacterial killing agents alone, binary combinations of alcohol and saturated or substantially saturated halide salts, such as sodium chloride and ternary combinations thereof containing urea are surprisingly and synergistically effective against a wide range of micro-organisms. Such binary and ternary combinations containing alcohol, saturated sodium chloride and optionally urea have been discovered to be simple and rapid decontaminating and sterilizing solutions for medical apparatus and particularly for soft contact lenses. Such compositions also have the advantage that they only contain materials endogenous to the human body.

Binary and ternary aqueous solutions according to the invention permit the effective and rapid sterilization; for example, typically in less than 12 hours, and more particularly in 8 hours, and as low as 4 or less hours; for example, 30 to 120 minutes, against the mixed micro-organisms normally associated with and which contaminate contact lenses. Once sterilization has been effected, the apparatus may be rinsed, e.g. in tap water, and, if desired, dried. The sterilizing solution may be used without damange to the lens or injury to the eye of the user. The invention permits the contacting of the contact lens with the solution containing as an active ingredient an alcohol in combination with a saturated or high-concentration salt solution for decontamination and sterilization purposes. The sterilizing solution is employed easily by contacting, such as by soaking overnight or for a short period of time; for example, 1 hour, or by washing, the lens to be sterilized with the aqueous sterilizing solution.

The sterilizing solution has been tested and has been found to be effective particularly against the five indicator organisms recommended by the Food and Drug Administration of the United States of America (the FDA) for evaluation of lens-sterilizing and -decontaminating solutions. In its simplest embodiment, the

0001888

06/A/260

sterilizing solution comprises binary combinations of a saturated or substantially saturated sodium chloride solution in combination with ethyl alcohol, and, optionally, where additional possible cleaning and some protein-denaturization is required, urea may be added.

The physiologically acceptable salt is preferably an inorganic salt and may be, for example, an alkali metal- or an alkaline earth metal-halide or sulphate. Thus the salt may be a sodium, potassium, lithium, calcium, magnesium or strontium chloride or bromide. The salt should, of course, have a reasonable degree of water solubility in the presence of the other component or components. The preferred salt is sodium chloride. The salt is preferably present in a concentration which is hypertonic with respect to the various organisms, for example, bacteria and fungi, which it is desired to kill. Thus the salt; for example, sodium chloride, may represent from about 2% to 33%, preferably from 5% to 33%, more preferably from 10% to 33%, and most preferably from 20% to 33% by weight of the composition.

The sterilizing solution includes, as the preferred embodiment, saturated solutions of sodium chloride. However, it is recognized that other non-toxic salts, such as those of alkali, including ammonium, halides, may be employed. However, sodium chloride is the preferred salt, due to its high sterilizing activity and wide acceptance, non-toxic nature and low cost.

The physiologically acceptable alcohol may be a lower, and preferably a lower monohydric alkanol for example containing up to 6 carbon atoms, e.g. methanol, isopropanol, butanol or preferably ethanol. The alcohol may represent from about 5% to 70%, preferably 10% to 50% and more preferably from 10% to 30% by volume of the composition.

The preferred alcohol is ethyl alcohol. However, other alcohol or alcohol-containing materials also may be employed, particularly the lower molecular-weight non-toxic alcohols, e.g. polyhydroxy alcohols, such as polyethylene glycol or a polyvinyl alcohol.

The physiologically acceptable protein-solubilizing agent is preferably such that it will split and denature the proteins likely to contaminate the object to be treated. Thus in the case of contact lenses, it is preferred to use an agent capable of denaturing and solubilizing the mucous protein associated with the eye. A particular example of a suitable solubilizing agent is urea. The solubilizing agent may be present in the composition at a concentration of from 2 to 8, and preferably from 5 to 8, molar.

The water used in the composition may be hard or soft and is preferably sterile-distilled or deionized water.

Preferred sterilizing solutions of the invention comprise the binary combinations of 25.8% weight/volume sodium chloride and 10% by volume ethyl alcohol; and 26.4% weight/volume sodium chloride and 20% by volume ethyl alcohol. Effective ternary solutions comprise 7.65% weight/ volume sodium chloride, 20% by volume ethyl alcohol, and about 2.90 molar urea; and 8.29% weight/volume sodium chloride, 2.90 molar urea and 10% by volume ethyl alcohol. The most particularly preferred

sterilizing solution comprises saturated sodium chloride, 2.00 molar urea and 20% by volume ethyl alcohol.

As will be seen from the above description, the compositions according to the invention fall into four general groups:

1. Aqueous mixtures of the salt and the alcohol;

2. Aqueous mixtures of the salt and the solubilizing agent;

3. Aqueous mixtures of the alcohol and the solubilizing agent; and

4. Aqueous mixtures of the alcohol, the solubilizing agent and the salt.

Of these groups 1 and 4 are preferred.

Particularly preferred are aqueous mixtures of ethanol and sodium chloride and especially aqueous ethanolic solutions which are substantially saturated with sodium chloride. Specific compositions from such groups include, but are not limited to:

| | |
|---|---|
| 16.6% w/v NaCl | 10% v/v Ethanol |
| 26.4% " " | 20% " " |
| 8.33% " " | 10% " " |
| 11.1% " " | 10% " " |
| 16.6% " " | 20% " " |
| 11.1% " " | 6.67% v/v " |
| 20.2% " " | 6.67% " " |
| 25.8% " " | 10% v/v " |

In group 4 preferred compositions comprise a mixture of sodium chloride, ethanol and urea. Particularly preferred are compositions saturated with sodium chloride. Specific compositions from group 4 include, but are not limited to:

| Urea (Molar) | NaCl % w/v | Ethanol % v/v |
|---|---|---|
| 2.67 | 11 | 6.67 |
| 2.90 | 7.65 | 20 |
| 2.67 | 11.1 | 10 |
| 2 | 11.1 | 6.67 |
| 2 | 8.33 | 6.67 |
| 2.67 | 8.33 | 10 |
| 2 | 8.33 | 10 |
| 2.67 | 8.33 | 6.67 |
| 2 | 11.1 | 10 |
| 2.9 | 8.29 | 10 |
| 2 | 7 | 6.67 |
| 2 | 8.3 | 20 |
| 2.67 | 11.1 | 20 |
| 2.67 | 8.3 | 20 |

In addition to the components set out above, the compositions of the invention may contain other physiologically acceptable ingredients; for example, compositions for use with contact lenses may contain ingredients which are acceptable or useful in the eye; for example, opthalmic topical medications that are compatible with (for example, do not precipitate in) the chosen solution.

The concentration of the ingredients in the sterilizing solution may vary depending on the object to be treated and the particular organisms to be removed. Thus the amount of salt employed is preferably sufficient to provide a saturated or substantially saturated solution, which amount of salt by weight will vary, depending upon the amount of alcohol present, with, of course, some variations based on the temperature. Typical concentrations of salts in the sterilizing solutions range from as high as 33% by weight sodium chloride for a fully saturated solution, alone, and typically to as low as about 5%, depending upon the amount of alcohol present, with typically the alcohol varying in amount by volume from 5% to 70%, preferably 10% to 50% and more typically between 10% to 25%. The amount of urea may vary as desired, ranging up to 8 molar, but more typically ranging from about 1 molar to about 3 molar.

The sterilizing solutions are easily prepared merely by admixing the ingredients to form a sterile, aqueous, clear solution. A preferred process is to mix ethanol and a saturated aqueous solution of sodium chloride. Of course, if desired and optionally, and apart from the active ingredients specified above, other components may be added, such as other microbial agents, buffers, lubricants, quaternary-ammonium compounds, viscosity-control agents, surfactants, wetting agents and similar active ingredients, provided that such ingredients do not alter or affect the sterilizing functions of the active ingredients.

The sterilizing solutions may be used in the treatment of soft contact lenses containing or contaminated with mixed microbial agents, and, therefore, the term 'sterilization' as used herein is directed to the sterilization of those five indicating organisms recommended by

the FDA and as tested herein for evaluation of lens-decontaminating solutions. Effective sterilization times by the solutions are measured by the hours required for total kill of the organisms, i.e. the number of hours required to obtain no survivors where the sensitivity of the assay is 10 cells per ml or less. The sterilizing solution may be used for 12 hours, (e.g. overnight storage of a lens in the solution) and more particularly for 6 hours and even more particularly for 60 minutes.

The method of use of the sterilizing solution is simple in that the wearer of the lens merely removes the lens from his eye and places it in a container with a sufficient amount of the sterilizing composition. The lens is allowed to soak for a period of at least about 30 minutes, and preferably 1 to 8 hours. This contacting of the lens may also be used to inhibit the formation of proteinaceous deposits and accumulations on the lens in use. Typically, the method of use is carried out at ambient temperatures, or at slightly elevated temperatures, if desired; for example, $20^{o}C$ to $40^{o}C$.

Hard contact lenses and other medical apparatus may be sterilized by washing or immersing the apparatus in the sterilizing solution. Such other medical apparatus may include, but not be limited to: catheters, tubes, surgical instruments and the like. The sterilizing solution may be used on medical apparatus and materials which, like the soft contact lenses, do not tolerate readily existing or harsh sterilizing methods, such as boiling, and more particularly wherein the medical apparatus and material easily exchange any liquid contained therein with a sterilizing solution.

The invention is illustrated, but in no way limited by the following Examples in which EtOH represents ethanol.

Example 1

Tests were carried out to determine the sterilizing effects of selected solutions of salt and/or urea and/or ethyl alcohol on micro-organisms. For these experiments, the final mix (cell suspension plus test solution) was designed to contain approximately $1 \times 10^6$ cells per ml. Test systems were incubated at room temperature ($22^\circ$C to $25^\circ$C). Aliquots were withdrawn at designated intervals of time, as shown in the Tables, and were serially diluted in sterile 0.9% saline solution. Duplicate pour plates were made at dilutions of $10^{-1}$, $10^{-3}$ and $10^{-5}$ for each sample, adding about 15 ml of cooled ($45^\circ$C) Trypticase Soy Agar to 1 ml of diluted sample in a sterile Petri dish. Colonies were counted after 48 hours incubation at $37^\circ$C.

Seven combinations were selected for study of their killing capacity against each of the five organisms recommended by the FDA for the evaluation of decontaminating solutions for contact lenses. The results of such experiments are shown in Table I.

TABLE I

Inactivation of Microbial Suspensions by Binary and Ternary Combinations

of Ethyl Alcohol, Sodium Chloride and Urea

| Organism | Exposure Time (hours) | | | | | | |
|---|---|---|---|---|---|---|---|
| 7.85% NaCl, 2.85M Urea | 0 | 0.25 | 0.5 | 1 | 2 | 4 | 6 |
| Candida albicans | $7.0 \times 10^5$ | $3.0 \times 10^5$ | $1.0 \times 10^5$ | $8.0 \times 10^4$ | $5.0 \times 10^4$ | 165 | --* |
| Serratia marcescens | $1.3 \times 10^6$ | $2.1 \times 10^4$ | $1.8 \times 10^3$ | $1.0 \times 10^2$ | ---* | ---* | ---* |
| Pseudomonas aeruginosa | $1.0 \times 10^6$ | $1.4 \times 10^5$ | $3.5 \times 10^2$ | ---* | ---* | ---* | ---* |
| Staphylococcus epidermidis | $2.0 \times 10^6$ | $6.0 \times 10^5$ | $5.2 \times 10^4$ | $1.3 \times 10^3$ | $2.4 \times 10^2$ | ---* | ---* |
| Aspergillus fumigatus | $1.9 \times 10^4$ | $3.0 \times 10^3$ | 29 | 2 | ---* | ---* | ---* |
| 7M Urea, 10% EtOH | | | | | | | |
| Candida albicans | $7.0 \times 10^5$ | $2.7 \times 10^4$ | $1.8 \times 10^2$ | $1.0 \times 10^2$ | ---* | ---* | ---* |
| Serratia marcescens | $1.3 \times 10^6$ | ---* | ---* | ---* | ---* | ---* | ---* |
| Pseudomonas aeruginosa | $1.0 \times 10^6$ | ---* | ---* | ---* | ---* | ---* | ---* |
| Staphylococcus epidermidis | $2.0 \times 10^6$ | ---* | ---* | ---* | ---* | ---* | ---* |
| Aspergillus fumigatus | $1.9 \times 10^4$ | 4 | ---* | ---* | ---* | ---* | ---* |

* Less than $1 \times 10^1$/ml

TABLE I (Ct'd)

Organism          Exposure Time (hours)

| 6.5M Urea, 6.67% EtOH | 0 | 0.25 | 0.5 | 1 | 2 | 4 | 6 |
|---|---|---|---|---|---|---|---|
| Candida albicans | $1.8 \times 10^6$ | $7.2 \times 10^4$ | $8.2 \times 10^3$ | $2.6 \times 10^3$ | ---* | ---* | ---* |
| Serratia marcescens | $2.0 \times 10^6$ | ---* | ---* | ---* | ---* | ---* | ---* |
| Pseudomonas aeruginosa | $1.6 \times 10^6$ | ---* | ---* | ---* | ---* | ---* | ---* |
| Staphylococcus epidermidis | $1.3 \times 10^6$ | $3.9 \times 10^4$ | $1.4 \times 10^4$ | ---* | ---* | ---* | ---* |
| Aspergillus fumigatus | $7.0 \times 10^4$ | 53 | 2 | ---* | ---* | ---* | ---* |
| **8.3% NaCl, 2.67M Urea 20% EtOH** | | | | | | | |
| Candida albicans | $1.8 \times 10^6$ | ---* | ---* | ---* | ---* | ---* | ---* |
| Serratia marcescens | $2.0 \times 10^6$ | ---* | ---* | ---* | ---* | ---* | ---* |
| Pseudomonas aeruginosa | $1.6 \times 10^6$ | ---* | ---* | ---* | ---* | ---* | ---* |
| Staphylococcus epidermidis | $1.3 \times 10^6$ | $7.3 \times 10^2$ | ---* | ---* | ---* | ---* | ---* |
| Aspergillus fumigatus | $7.0 \times 10^4$ | ---* | ---* | ---* | ---* | ---* | ---* |

* Less than $1 \times 10^1$/ml

TABLE I (Ct'd)

Organism                                    Exposure Time (hours)

| 25.8% NaCl, 10% EtOH | 0 | 0.25 | 0.5 | 1 | 2 | 4 | 6 |
|---|---|---|---|---|---|---|---|
| Candida albicans | $1.1 \times 10^6$ | $1.6 \times 10^2$ | ---* | ---* | ---* | ---* | ---* |
| Serratia marcescens | $1.5 \times 10^6$ | 89 | ---* | ---* | ---* | ---* | ---* |
| Pseudomonas aeruginosa | $8.0 \times 10^5$ | ---* | ---* | ---* | ---* | ---* | ---* |
| Staphylococcus epidermidis | $2.4 \times 10^6$ | $2.4 \times 10^2$ | ---* | ---* | ---* | ---* | ---* |
| Aspergillus fumigatus | $3.0 \times 10^4$ | ---* | ---* | ---* | ---* | ---* | ---* |
| **20.2% NaCl, 6.67% EtOH** | | | | | | | |
| Candida albicans | $1.1 \times 10^6$ | $4.2 \times 10^3$ | $1.1 \times 10^3$ | ---* | ---* | ---* | ---* |
| Serratia marcescens | $1.5 \times 10^6$ | $1.0 \times 10^5$ | $8.3 \times 10^3$ | $6.6.x \times 10^2$ | ---* | ---* | ---* |
| Pseudomonas aeruginosa | $8.0 \times 10^5$ | ---* | ---* | ---* | ---* | ---* | ---* |
| Staphylococcus epidermidis | $2.4 \times 10^6$ | $3.2 \times 10^5$ | $2.0 \times 10^4$ | ---* | ---* | ---* | ---* |
| Aspergillus fumigatus | $3.0 \times 10^4$ | $3.1 \times 10^3$ | $1.5 \times 10^2$ | $1.0 \times 10^2$ | $10 \times 10^2$ | 10 | ---* |

* Less than $1 \times 10^1$/ml

## TABLE I (Ct'd)

| Organism | Exposure Time (hours) | | | | | | |
|---|---|---|---|---|---|---|---|
| 7.65% NaCl, 2.90M Urea, 20% EtOH | 0 | 0.25 | 0.5 | 1 | 2 | 4 | 6 |
| Candida albicans | $1.8 \times 10^6$ | ---* | ---* | ---* | ---* | ---* | ---* |
| Serratia marcescens | $2.0 \times 10^6$ | ---* | ---* | ---* | ---* | ---* | ---* |
| Pseudomonas aeruginosa | $1.6 \times 10^6$ | ---* | ---* | ---* | ---* | ---* | ---* |
| Staphylococcus epidermidis | $1.3 \times 10^6$ | $6.8 \times 10^2$ | ---* | ---* | ---* | ---* | ---* |
| Aspergillus fumigatus | $7.0 \times 10^4$ | ---* | ---* | ---* | ---* | ---* | ---* |

* Less than $1 \times 10^1$/ml

The maximum concentration of sodium chloride and urea in aqueous solutions (no ethyl alcohol) is the least effective combination in this series of tests. The most effective are 8.3% sodium chloride, 2.67M urea, 20% ethyl alcohol and 7.65% sodium chloride, 2.90M urea, 20% ethyl alcohol, both of which completely killed four of the indicator strains by 0.25 hour and reduced the titer of Staphylococcus epidermidis by more than 3 logs by 0.25 hour, with total kill of this strain by 0.5 hour.

Example 2

Testing of selected combinations against mixed microbial suspensions

Three combinations, a binary (25.8% sodium chloride, 10% ethyl alcohol) and two ternaries (8.29% sodium chloride, 2.90M urea, 10% ethyl alcohol; and 7.65% sodium chloride, 2.90M urea, 20% ethyl alcohol), were selected for evaluation against mixed microbial suspensions of the five indicator organisms. For all experiments, the final mix (cell suspension plus test solution) was designed to contain approximately $1 \times 10^6$ cells per ml. Sterile 0.9% (physiologic) saline was used as a control. Test systems were incubated at room temperature ($22^\circ$C to $25^\circ$C). Aliquots were withdrawn at designated intervals of time, as shown in the Tables, and were serially diluted in sterile 0.9% saline. Duplicate pour plates were made at dilutions of $10^{-1}$, $10^{-3}$ and $10^{-5}$ for each sample, adding about 15 ml of cooled ($45^\circ$C) Trypticase Soy Agar to 1 ml of diluted sample in a sterile Petri dish. Colonies were counted after 48 hours of incubation of $37^\circ$C. The results are shown in Table II.

TABLE II

Inactivation of a Mixed Microbial Suspension* by Binary and Ternary Combinations
of Ethyl Alcohol, Sodium Chloride and Urea

Test Solution | | | | Exposure Time | | | |

| Test Solution | 0 hr | 0.25 hr | 0.5 hr | 1 hr | 2 hr | 4 hr | 6 hr |
|---|---|---|---|---|---|---|---|
| Control (0.9%NaCl) | $1.3 \times 10^6$ | $1.0 \times 10^6$ | $1.4 \times 10^6$ | $9.3 \times 10^5$ | $1.1 \times 10^6$ | $1.5 \times 10^6$ | $1.7 \times 10^6$ |
| 8.29% NaCl, 2.90M Urea, 10% EtOH | $1.3 \times 10^6$ | $3.8 \times 10^1$ | 3 | ---** | ---** | ---** | ---** |
| 7.65% NaCl, 2.90M Urea, 20% EtOH | $1.3 \times 10^6$ | $6.9 \times 10^2$ | $3.8 \times 10^2$ | ---** | ---** | ---** | ---** |
| 25.8% NaCl, 10% EtOH | $1.3 \times 10^6$ | $3.1 \times 10^5$ | $6.0 \times 10^4$ | ---** | ---** | ---** | ---** |

\* Serratia marcescens, Pseudomonas aeruginonsa, Staphylococcus epidermidis, Candida albicans,
Aspergillus fumigatus (each at a concentration of $>10^5$/ml)

\*\* Less than $1 \times 10^1$/ml

The ternary combination of 8.29% NaCl, 2.90M urea, 10% EtOH is the most rapidly effective antimicrobial solution of the three tested. Nonetheless, all three combinations resulted in total elimination of viable micro-organisms within one hour.

Example 3

Tests were carried out to evaluate the efficacy of the selected sterilizing solution on soft contact hydrogel-type lenses seeded with known levels of bacteria.

Inoculation of lenses  A series of sterile, flexible, soft contact lenses (Corneal Sciences Inc) (decontaminated by boiling in physiological saline) was drained (using sterile cotton swabs for handling) and seeded (on the inner concave surface) with 5 $\mu$l of a suspension of the organisms set out in Table II (1 x $10^6$ cells), and was allowed to dry at room temperature in a covered sterile Petri dish or covered multi-well plates for 0, 5, 10, 15 and 30 minutes. At the end of each of these time periods, a seeded lens was transferred with sterile forceps to a tube of sterile nutrient broth which was incubated at 37$^O$C for 6 hours. Growth in the broth culture was estimated by optical density every 2 hours. The maximum drying time which allows the best growth, equal to the zero-drying-time control, was selected as a drying time to use in subsequent studies. Optimally, the time chosen should be adequate to load the lens with micro-organisms, but not so long that the inoculum is killed by the drying.

Decontamination of seeded lenses  A series of lenses (flexible soft contact lenses, Corneal Sciences Inc) was seeded with bacterial suspensions (1 x 10$^6$ cells in 1 ml, same five species as in Example 2) and was allowed to dry for 10 minutes.

Single lenses, contained in the well of a multi-well plate, were filled with 1.0 ml of the test-decontaminating solutions. At 0, 0.25, 0.5, 1, 2, 4 and 6 hours, lenses were drained and transferred to a tube containing 5 ml of sterile physiological saline. After thorough agitation, the saline was serially diluted and bacterial counts were determined by the pour-plate method. The lenses were transferred to a broth tube and were incubated for 48 hours as a further sterility check. The spent decontaminating solutions were serially diluted from a starting dilution of 1:5, and pour plates were made to determine actual numbers of survivors in the solutions.

Flexsol (a trade mark of Burton Parsons Co) is a sterile, buffered, isotonic solution of sodium chloride, sodium borate, boric acid, polyvinylpyrrolidone, polyoxyethylene and polyoxypropylene, preserved with Thiomersal 0.001%, chlorhexidene 0.005% and disodium edetate 0.1%.

The results are shown in Table III.

TABLE III

Decontamination of Soft Contact Lenses Seeded with a Mixed Microbial Suspension by Binary and Ternary Combinations of Ethyl Alcohol, Sodium Chloride and Urea and by 'Flexsol'

25.8% NaCl, 10% EtOH

| | 0 hr | 0.25 hr | 0.5 hr | 1 hr | 2 hr | 3 hr | 4 hr | 6 hr |
|---|---|---|---|---|---|---|---|---|
| 'Spent' solution* | $8 \times 10^5$ | $4 \times 10^5$ | $5 \times 10^4$ | 300 | 200 | 165 | 100 | 55 |
| Lens count** | >500 | >500 | 315 | 190 | 50 | 35 | 40 | 22 |
| Sterility check | ++ | ++ | ++ | + | + | + | + | + |

7.65% NaCl, 2.90M Urea, 20% EtOH

| | 0 hr | 0.25 hr | 0.5 hr | 1 hr | 2 hr | 3 hr | 4 hr | 6 hr |
|---|---|---|---|---|---|---|---|---|
| 'Spent' solution* | $1 \times 10^6$ | $8 \times 10^5$ | 5 | 0 | 0 | 0 | 0 | 0 |
| Lens count** | >500 | 180 | 20 | 5 | 0 | 0 | 0 | 0 |
| Sterility check | ++ | + | + | - | - | - | - | - |

\* 'Spent' solution data are in numbers of bacteria/ml
\*\* Lens count data are in actual numbers of bacteria on lens

## TABLE III (Ct'd)

8.29% NaCl, 2.9M Urea, 10% EtOH

|  | 0 hr | 0.25 hr | 0.5 hr | 1 hr | 2 hr | 3 hr | 4 hr | 6 hr |
|---|---|---|---|---|---|---|---|---|
| 'Spent' solution* | $1 \times 10^6$ | $3 \times 10^3$ | $2 \times 10^3$ | 35 | 15 | 0 | 0 | 0 |
| Lens count** | >500 | 75 | 69 | 26 | 9 | 0 | 0 | 0 |
| Sterility check | ++ | + | + | + | − | − | − | − |
| 'Flexsol' | | | | | | | | |
| 'Spent' solution* | $1 \times 10^6$ | $6 \times 10^3$ | 50 | 35 | 20 | 0 | 0 | 0 |
| Lens count** | >500 | 81 | 55 | 16 | 12 | 0 | 5 | 0 |
| Sterility check | ++ | + | + | + | + | − | − | − |
| 26.4% NaCl, 20% EtOH | | | | | | | | |
| 'Spent' solution* | $9 \times 10^5$ | $5 \times 10^3$ | 25 | 0 | 0 | 0 | 0 | 0 |
| Lens count** | 500 | 150 | 10 | 0 | 0 | 0 | 0 | 0 |
| Sterility check | ++ | + | − | − | − | − | − | − |

** Lens count data are in actual numbers of bacteria on lens, not bacteria/ml in wash diluent

* 'Spent' solution data are in numbers of bacteria/ml

08/N/262

The binary combination of 26.4% NaCl and 20% EtOH is the most effective decontaminating solution of the five tested. It is noteworthy that Flexsol, the only FDA-approved decontaminating solution for soft contact lenses, does not achieve dependable sterilization of the soft contact lens tested within four hours, in contrast to only one hour for the binary combination of 26.4% NaCl and 20% EtOH in aqueous solutions.

Claims

1.  An aqueous sterilizing solution characterised in that the solution comprises, as active ingredients in combination, a physiologically acceptable salt in a concentration of at least 5% w/w and a physiologically acceptable alcohol.

2.  A solution according to Claim 1, characterised in that the salt is sodium chloride.

3.  A solution according to Claim 1, or Claim 2 characterised in that the alcohol is ethanol.

4.  A solution according to any one of the preceding claims, characterised in that it comprises from about 5 to 33 weight percent of the salt.

5.  A solution according to any one of the preceding claims, characterised in that it comprises from about 5% to 70% by volume of the alcohol.

6.  A solution according to any one of the preceding claims, characterised in that the salt comprises from about 20% to 30% by weight of sodium chloride, and the alcohol comprises from about 10% to 20% by volume of ethanol.

7.  A solution according to any one of the preceding claims, characterised in that it includes a water-soluble, physiologically acceptable, protein-denaturing agent.

8.  A solution according to Claim 7, characterised in that the protein denaturing agent is urea.

9.  A solution according to Claim 8, characterised in that urea is present in a molar concentration of from about 2.9 to 3 molar.

10. A solution according to any one of Claims 1 to 6, characterised in that it consists essentially of sodium chloride, ethanol and water.

11. A solution according to any one of Claims 1 to 9, characterised in that it consists essentially of sodium chloride, ethanol, water and urea.

12. A solution according to any one of the preceding claims characterised in that it is substantially saturated with respect to the salt.

13. A method of sterilizing an object, characterised by contacting, the object, for a time sufficient to effect sterilization, with a sufficient amount of a solution which comprises a sterilizing mixture of a physiologically acceptable salt and a physiologically acceptable alcohol.

14. A method according to Claim 14, characterised in that the time is from about 30 minutes to 12 hours.

15. A method according to Claim 13 or Claim 14, characterised in that the object is medical apparatus.

16. A method according to any one of Claims 13 to 15, characterised in that the apparatus to be sterilized is a contact lens.

17. A method according to Claim 16, characterised in that the lens comprises a flexible, hydrogel, soft contact lens of the hydrophilic type.

18. A method according to any one of Claims 13 to 17, characterised

0001888

12/A/262

in that the sterilizing composition comprises a composition according to any one of Claims 1 to 12.

19. A method according to any one of Claims 13 to 18, characterised in that the object is composed of a water-containing polymeric material capable of the exchange of water with the sterilizing solution.

20. A method of making an aqueous sterilising solution characterised in that a physiologically acceptable salt and a physiologically acceptable alcohol are mixed in sterilising proportions.

0001888

European Patent Office

**EUROPEAN SEARCH REPORT**

Application number

EP 78 30 0478

## DOCUMENTS CONSIDERED TO BE RELEVANT

| Category | Citation of document with indication, where appropriate, of relevant passages | Relevant to claim |
|---|---|---|
| A | US - A - 3 873 696 (K.J. RANDERI) <br> * Column 2, lines 51-62 * | 1,2,13-19 |
| A | FR - A - 2 226 677 (ALLERGAN) <br> * Page 15, lines 23-30 * | 1,7 |

**CLASSIFICATION OF THE APPLICATION (Int. Cl.³)**

A 61 L 13/00
1/00

**TECHNICAL FIELDS SEARCHED (Int.Cl.²)**

A 61 L 13/00
A 61 K 33/14
A 61 L  1/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 01-02-1979 | PELTRE |

EPO Form 1503.1  06.78